# EUROPEAN PATENT SPECIFICATION

(11) **EP 0 930 370 B1**
(45) Date of publication and mention of the grant of the patent: **08.08.2007**
(21) Application number: 98123569.0
(22) Date of filing: 10.12.1998
(51) Int. Cl.: C12Q 1/68

(54) **Labeled primer for use in detection of target nucleic acids**
Markierter Primer, geeignet für die Detektion von Nukleinsäuren
Amorce marquée approprié pour la détéction des acides nucléiques

(30) Priority: 15.12.1997 DE 19755642; 19.02.1998 DE 19806850; 03.08.1998 DE 19834913
(43) Date of publication of application: 21.07.1999
(60) Divisional application: 06000965.1
(73) Proprietor: CSL Behring GmbH, 35041 Marburg (DE)
(72) Inventor: Weimer, Thomas Dr., 35075 Gladenbach (DE)

(56) References cited:
- WO-A-90/12115
- WO-A-97/29210
- US-A- 5 804 375

## Description

The invention relates to a labeled primer for nucleic acid amplification reactions (for example the polymerase chain reaction) and to a process for detecting a DNA sequence by means of a nucleic acid amplification process in which a labeled primer is used.

As is known, the polymerase chain reaction (PCR) is a very elective method for detecting small quantities of a known nucleic acid sequence in a sample (Erlich HA. Gelfand, D. Sninsky J.J. (1991). Science. 252, pp. 1643-1651 which publication is incorporated herein by reference; PCR Protocols. Current methods and applications (1993) edited by B.A. White, Humana Press, Totowa, New Jersey, ISBN 0-89603-244-2 which publication is incorporated herein by reference). If the sequence of, for example, a viral DNA is already known, it is possible to synthesize a pair of primers which are complementary to regions on opposite single strands and which flank the sought-after DNA sequence. Under PCR conditions, which are known per se, a sequence of normally more than 30 reaction cycles can then be used to amplify large quantifies of a specific DNA in vitro. The PCR cycles amplify a DNA fragment, which is of a specific size and which is composed of the lengths of the two primers plus the length of the e.g. viral DNA between them, when the sought-after e.g. viral DNA is present in the sample. The PCR technique is so sensitive that it can be used to detect extraordinarily small quantities of a DNA with a high degree of reliability.

International Patent Application WO 92/02638 herein incorporated by reference discloses a process for detecting a DNA sequence, in which process a sample, which contains or is suspected to contain the DNA to be detected (as a single strand) i.e. the target DNA. is hybridized with two different primers, i.e. the forward primer and the reverse primer, which flank the DNA strand to be amplified i.e. the target DNA. A labeled oligonucleotide probe, which is provided in a preferred embodiment of WO 92/02638 with a fluorescent dye system as label both at its 5' end and at its 3' end, is also employed in the reaction. This labeled probe is selected such that it hybridizes between the two primers within the DNA segment to be amplified i.e. within the target DNA. The two fluorescent dyes which are attached to the probe as preferred label have the characteristic feature that the fluorescence of one of the dyes, the reporter dye, is decreased ("quenched") by the proximity of the second molecule, i.e. the quencher by a process known as fluorescence resonance energy transfer (Stryer, L 1978; Fluorescence energy transfer as a spectroscopic ruler. Ann. Rev. Biochem. 47: 819-846, which is incorporated herein by reference). This labeled probe, which binds to the DNA between the two primers, which are characterized by the abovementioned sequences, has, as an example, the following sequence:

### ⁵FAM-TGG TGG TCT GGG ATG AAG GTA TTA TT-TAMRA^{3'}

A sequence of this nature can be ordered and obtained from a number of companies and is intended for use in a 5'-nuclease assay, i.e. the TaqMan^{®} assay. The method above is described in detail by Livak K.J., Flood S.J.A., Marmaro J., Giusti W., Deetz K., Oligonucleotides with fluorescent dyes at opposite ends provide a quenched probe system useful for detecting PCR product and nucleic acid hybridization. PCR Method and Appl. 1995; 4:357-362 which publication is incorporated herein by reference.

The special feature of this probe is that the fluorescence of the dye (FAM), i.e. the reporter dye, which is attached to the 5' end of the probe is reduced by the proximity of the second fluorescent dye (TAMRA), i.e. the quencher dye, which is arranged at the 3' end of the probe, see above.

If the new DNA strand is now formed, during the course of the amplification and under the influence of a suitable, preferably thermostable DNA polymerase, e.g. the TaqDNA polymerase not only displaces the labeled probe from the single strand but also, by means of its 5'→3' nuclease activity, degrades the probe and thereby releases the two fluorescent dyes. The fluorescence of the reporter dye is now no longer suppressed by the quencher dye and increases. If a fluorescence spectrometer is now used to measure the fluorescence at the wavelength of the reporter dye, it is then possible to observe an increase in fluorescence which corresponds to the quantity of newly formed DNA.

The fact that a labeled probe is required, in addition to the forward primer and the reverse primer, in order to be able to observe or measure the amplification of the DNA segment to be detected has to be regarded as a disadvantage of this method. The object of simplifying this known process therefore arose.

it has now been found that there is no need to use an additional labeled probe in the polymerase chain reaction if at least one of the two primers is labeled with e.g. in a preferred embodiment of the invention a reporter molecule and a quencher molecule (in generalized terms: an interactive label system) and, at the same time, care is taken to ensure that the primer which is labeled does not hybridize completely with the DNA strand to be amplified.

The invention therefore uses a labeled primer for nucleic acid amplification reactions (for example the polymerase chain reaction), which primer is labeled, in a preferred embodiment with a label system at or near the 3' end of the primer, with in the preferred embodiment a reporter dye molecule and a quencher molecule, and at least one, preferably at least the last two to five, or more, bases at the 3' end of the primer (i.e. a 3' terminal deliberately mis-matched portion) which is labeled in this way are not complementary to the DNA sequence to be amplified. The label or part of a label system is attached to the 3' terminal mismatched portion, preferably to the 3' end nucleotide. The length of the unpaired region is selected and/or optimized known to the man of the art so as to the particular label and particular polymerase used. Such a labeled primer is not able to undergo complete base pairing at its 3'-end with the DNA sequence to be amplified. Under the influence of the polymerase employed for the amplification, which polymerase must possess proof-reading properties, the unpaired bases of the labeled primer, together with the label used, e.g. the reporter dye molecule or quencher molecule as may be the case, are released by the 3'→5' nucleolytic activity of the polymerase before the actual elongation reaction takes place. This, however, results in the examplified case that the quencher is no longer being in spatial proximity to the reporter dye, whose fluorescence therefore increases, thus indicating presence of a target nucleic acid and/or target nucleic acid amplification.

The invention therefore also relates to a process for detecting a DNA target nucleic acid by means of nucleic acid amplification, in which process one of the primers possesses the abovementioned features. In the amplification, for which it is possible to use one or more thermostable DNA polymerases, at least one of which must also have proof-reading properties, the unpaired bases of the labeled primer, together with the label or part of a label system e.g. reporter dye which is attached to it, are then released, resulting in a signal increase, e.g. fluorescence increasing at the wavelength of the reporter dye.

In the novel process, the forward primer or the reverse primer or both can be labeled with the reporter and quencher molecules each as described above. While the quencher dye is liberated into the reaction solution, the newly formed DNA segment also carries the fluorescent reporter dye in addition to the remainder of the primer. This is the preferred procedure for quantitative applications. However, the labeling with quencher and reporter dye can also be effected equally well in the converse manner, so that the reporter dye is liberated into the solution and the newly formed DNA segment carries the quencher. If several parameters are to be simultaneously detected in parallel (multiplexing), it is then expedient to select reporter dyes which can be detected in parallel. It is disclosed here that the method of the invention is very well suited for multiplexing.

In a generalized way the 3'-end of a primer is labeled or attached to the part of a label system, as described below, by incorporating moieties detectable by spectroscopic, photochemical, immunochemical, or chemical means. The method of linking or conjugating the label to the primer depends, of course, on the type of label(s) used and the position of the label on the primer.

A variety of labels that would be appropriate for use in the invention, as well as methods for their inclusion in the primer, are known in the art and include, but are not limited to, enzymes (e.g., alkaline phosphatase and horseradish peroxidase) and enzyme substrates, radioactive atoms, fluorescent dyes, chromophores, chemiluminescent labels, electro-chemiluminescent labels, such as Origen^{™} (Igen), ligands having specific binding partners, or any other labels that may interact with each other to enhance, alter, or diminish a signal. Of course, should the amplification be made via PCR and be practiced using a thermal cycler instrument, the label must be able to survive the temperature cycling required in this automated process.

Among radioactive atoms, ³²P is preferred. Methods for introducing ³²P into nucleic acids are known in the art, and include, for example, 5' labeling with a kinase, or random insertion by nick translation. Enzymes are typically detected by their activity. "Specific binding partner" refers to a protein capable of binding a ligand monoclonal antibody specific therefor. Other specific binding partners include biotin and avidin or streptavidin, lgG and protein A, and the numerous receptor-ligand couples known in the art.

The above description is not meant to categorize the various labels into distinct classes, as the same label may serve in several different modes. For example, ¹²⁵I may serve as a radioactive label or as an electron-dense reagent. HRP may serve as enzyme or as antigen for a monoclonal antibody. Further, one may combine various labels for desired effect. For example, one might label a primer with biotin, and detect the presence of the primer with avidin label with ¹²⁵I, or with an anti-biotin monoclonal antibody labeld with HRP. Other permutations and possibilities will be readily apparent to those of ordinary skill in the art and are considered as equivalents within the scope of the instant invention.

Fluorophores for use as labels in constructing labeled primers of the invention include rhodamine and derivatives, such as Texas Red, fluorescein and derivatives, such as 5-bromomethyl fluorescein, Lucifer Yellow, IAEDANS, 7-Me₂N-coumarin-4-acetate, 7-OH-4-CH₃-coumarin-3-acetate, 7-NH₂-4-CH₃-coumarin-3-acetate (AMCA), monobromobimane, pyrene trisulfonates, such as Cascade Blue, and monobromotrimethylammoniobimane. In general, fluorophores with wide Stokes shifts are preferred, to allow using fluorimeters with filters rather than a monochromometer an to increase the efficiency of detection. However, the particular label(s) chosen preferably has (have) some quality (e.g. the reporter-quencher relationship) to allow detection in a homogeneous assay system.

Detection or verification of the label in the processes disclosed is accomplished by a variety of methods and is dependent on the source of the label(s) employed. In the preferred embodiment of the invention, the increase of fluorescence is measured in a suitable fluorometer.

In a preferred embodiment of the instant invention two interactive labels on a single primer are used as a reporter-quencher label system, see above and the examples.

Examples for reporter molecules are 6-carboxy-Fluorescein (FAM), Tetrachloro-6-carboxy-fluorescein (TET), 6-Carboxy-X-Carboxy-Tetramethyl-Rhodamin (ROX). Examples of quencher molecules are 6-Carboxy-Tetramethyl-Rhodamin (TAMRA) or 4-(4'-Dimethylamino-phenylazo) benzoic acid (DABCYL). Whereas TAMRA is a fluorescent dye, DABCYL is not.

Different DNA polymerases can be employed for the novel amplification reaction. If the DNA polymerase which is used has the proof-reading property, it is then possible to carry out the reaction using one single polymerase (e.g. ULTma^{®} DNA polymerase, which is produced for Perkin Elmer by Roche Molecular Systems, Branchburg, New Jersey, USA). Otherwise, it is necessary to use a mixture of several polymerases, with other polymerases having 3'→5' nuclease activity also being used in addition to, for example, Taq DNA polymerase. Tli DNA polymerase (Promega Corporation, Madison, Wl, USA) or Vent DNA polymerase (New England Biolabs, Beverly, MA, USA) are suitable thermostable polymerases possessing 3'-5' nuclease activity. Very well suited for the instant invention is the AccuTaq™ LA DNA Polymerase Mix sold by SIGMA Corp. (Annex 1). Such Mix and related enzyme compositions are described in U.S. Patent No. 5,436,149 in example 6 ibidem, which patent disclosure is herewith incorporated by reference in its entirety.

The novel processes are based on recognition of the fact that the unpaired bases of the primer are a point of attack for the polymerase which possesses a proof-reading function. The thermostable DNA polymerases which are employed possess a 3'→5' nuclease activity, preferably 3'→5' exonuclease activity which results in the nonhybridized bases, together with as the case may be, the quencher or reporter molecule, being released.

The primers used in the novel processes comprise preferably oligonucleotides of 18 to 25 bases in the paired or matching region as related to the target nucleic acid for average G/C and A/T ratios, but are somewhat longer in case of more A/T base pairings and conform to the general methodology disclosed above for PCR.

The processes disclosed are valuable, in particular, for their simplicity in comparison to e.g. WO 92/02638, because only two oligonucleotides (primers) are required for implementing the nucleic acid amplification, for example the PCR. This is advantageous when, for example, conserved nucleic acid segments have to be amplified for detecting variable target sequences, such as those of viruses, or several, e.g. viral, parameters are to be detected simultaneously.

In addition to this, the method which is described here is very well suited for quantitative amplification, since the increase in fluorescence is directly proportional to the quantity of amplified DNA.

The most preferred embodiments of the invention are processes using PCR as the nucleic acid amplification method and wherein the 3' mismatched primer is labeled by a quenchable fluorescent dye molecule and a quencher molecule where at least one of said molecules is at or within the mismatched 3' end of said primer, In case a RNA target is to be detected a reverse transcription of the target RNA into DNA is perfomed preceding amplification via PCR. In case the target nucleic acid is abundant, a single normal PCR along protocols known to the man of the art but using labeled primer is preferable (Example 2). In case high sensitivity of target detection is desired, a first PCR amplification amplifying the target using non-labeled, perfectly matching primers may precede a second amplification of the target nucleic acid via nested PCR using the labeled primers of the invention. In especially the second amplification (nested PCR) it has been found that the addition of unlabeled primer with or without the 3' mismatching portion or tail can be of advantage in that it may render the amplification more efficient and allows for reduction of the labeled primer.

Reagents employed in the methods of the invention can be packaged into diagnostic kits. Diagnostic kits include the labeled primers in separate containers. If the primer(s) is (are) unlabeled, the specific labeling reagents may also be included in the kit. The kit may also contain other suitably packaged reagents and materials needed for sample preparation and amplification, for example, extraction solutions for the target nucleic acid, buffers, dNTPs, and/or polymerizing means and for detection analysis, for example, enzymes and solid phase extractants, as well as instructions for conducting the assay. Another object of the invention is the various reaction mixtures for detecting a target nucleic acid in a sample useful for the processes disclosed. Samples may be body fluids of human or animal origin, or extracts of any body component of interest. Preferred samples are blood, plasma or any products resulting from same.

The invention is illustrated but not limited by the examples that follow:

### Example 1

This example is for applications which require highest sensitivity. In this case, a first round of amplification precedes the nested PCR which contains the labeled primer.

In order to detect hepatitis C virus RNA, it is first of all extracted using standard methods (for example Ishizawa M., Kobayashi Y, Miyamura T, Matsuma, S: Simple procedure of DNA isolation from human serum. Nucl. Acids Res. 1991; 19:5792 which publication is incorporated herein by reference.) and isolated RNA is subsequently reverse transcribed and amplified using standard methods (RT-PCR). The nested amplification and detection reaction is set up as follows:

5 µl of 10 x ULTma buffer (100 mM Tris-HCl, pH 8.8, 100 mM KCl, 0.02% Tween 20, from Perkin-Elmer, 7 µl of 25 mM MgCl₂, 8 µl of primer 1 (see SEQ ID No. 1 of the sequence listing) (10 pmol/µl), 4 µlof primer 2 (see SEQ ID No. 2 of the sequence listing) (10 pmol/µl), 0.25 µl of primer 3 (see SEQ ID No. 3 of the sequence listing) (10 pmol/µl), 2 µl of dNTPs (10 mM) and 0.5 µl of ULTma DNA polymerase possessing a proof-reading function (Perkin-Elmer, 6 units / µl) are added to 5 µl of the RT-PCR reaction, after which the whole is mixed with 18.25 µl of water and subjected to the following thermocycles:
1. initial denaturation for 1 minute at 90°
2. 35 cycles, of, in each case, 28 seconds at 94°C, for denaturation, and 1 minute at 56 °C, for annealing and extension
3. cooling at 4°C until evaluated.

The PCR reaction is evaluated in a fluorescence spectrometer. For this, the fluorescence is measured at the reporter wavelength (518 nm for FAM). A threshold value based on the fluorescence of negative controls which do not contain any target sequence is calculated, and used to evaluate unknown values.

### Example 2

This example is for applications which do not require highest sensitivity. In this case the single amplification reaction contains the labeled primer.

In order to amplify hepatitis B virus DNA from seropositive patients, it is first of all extracted using standard methods (for example Ishizawa M., Kobayashi Y., Miyamura T, Matsuma, S: Simple procedure of DNA isolation from human serum. Nucl. Acids Res. 1991; 19:5792). The amplification is set up as follows:

5 µl of 10 x ULTma buffer (100 mM Tris-HCl, pH 8.8, 100 mM KCl, 0.02% Tween 20, from Perkin-Elmer), 7 µl of 25 mM MgCl₂, 8 µl of primer 4 (see SEQ ID No. 4 of the sequence listing) (10 pmol/µl), 4 µl of primer 5 (see SEQ ID No. 5 of the sequence listing) (10 pmol/µl), 0.25 µl of primer 6 (see SEQ ID No. 6 of the sequence listing) (10 pmol/µl), 2 µl of dNTPs (10 mM) and 0.5 µl of ULTma DNA polymerase possessing a proof-reading function (Perkin-Elmer, 6 units / µl) are added to 5 µl of the extracted DNA (i.e. target nucleic acid), after which the whole is mixed with 18.25 µl of water and subjected to the following thermocycles:
1. initial denaturation for 1 minute at 90°
2. 35 cycles, of, in each case, 28 seconds at 94°C, for denaturation, and 1 minute at 58 °C, for annealing and extension
3. cooling at 4°C until evaluated.

Evaluation of results is done as described in example 1.
In both examples 1 and 2 the Reporter was FAM and the Quencher TAMRA.

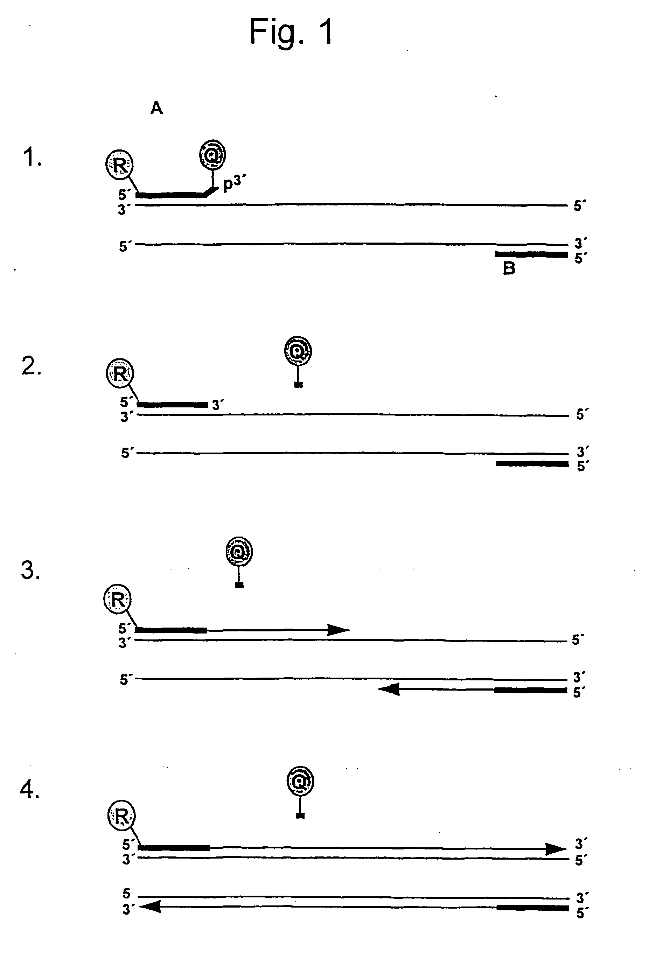

### Legend of Figure 1

The figure depicts a cycle of a claimed process ("TaqWoman Assay")
- A: denotes the forward primer with its unpaired 3' end
- B: denotes the reverse primer
- R: denotes the reporter molecule
- Q: denotes the quencher molecule

The so-called annealing takes place in 1., with the 3' end remaining unpaired since it is not complementary.

The so-called proof reading takes place in 2., with the enzyme possessing a proof-reading property correcting, i.e. removing, the unpaired 3' end.

The so-called strand elongation (elongation phase) takes place in 3., which phase has then finished in 4.

### ANNEX 1

| | | |
|---|---|---|
| **AccuTaq™ LA DNA POLYMERASE MIX*** | **D 8045** | 125 units |
| **For Long, Accurate PCR** | | 500 units |
| | | 1,500 units |

AccuTaq LA DNA Polymerase Mix is an optimized two-polymerase enzyme system for long, high-fidelity PCR. This mixture is an optimal blend of Sigma's high performance Taq DNA polymerase and a small amount of a polymerase with the 3'→5' exonuclease activity necessary for proofreading. This formulation achieves greater yields with higher fidelity (up to G.5x) than standard Taq DNA polymerase. PCR amplifications from 0.25 to more than 40 kb are possible. AccuTaq LA is ideally suited for amplifying complex DNA templates such as human genomic DNA. Its high fidelity makes it the enzyme of choice when performing amplifications where a low error frequency is critical, such as in RT-PCR and cloning.
- Greater fidelity (up to 6.5x) than standard Taq DNA polymerase.
- Amplifications up to 22 kb for complex DNA templates such as genomic DNA.
- Amplifications up to 40 kb for less complex templates such as plasmid DNA.
- More robust reactions and greater flexibility than single-enzyme high-fidelity systems.
- A vial of DMSO is included for enhancing amplification of complex templates such as genomic DNA.
- A detailed technical bulletin with amplification parameters for 1-40 kb lengths of DNA is provided. Supplied as 5 units/ul and used at 2.5 units/50µl reaction.
   Unit Definition: One unit incorporates 10 nmol of total dNTPs Into acid-precipitabfe DNA in 30 min. at 74°C.
   Storage: -20°C
   Shipped in wet ice
   R:22.36/37/38 S: 26.36-23
   Licensed under U.S. Patent No. 5.438.149 owned by Takara Shuzo Co., Ltd.

### TECHNICAL BULLETIN

### Introduction

The Polymerase Chain Reaction (PCR)† Is a powerful technique commonly used for molecular biology applications such as cloning, sequencing and genome mapping. The primary enzyme used in PCR is Taq DNA polymerase. Polymerase Chain Reaction using Taq DNA polymerase is generally limited to amplifications up to 5-kb. This is due in part because Taq DNA polymerase has no 3'→5' exonuclease or ''proofreading" activity, which means periodic misincorporations are not repaired. After a misincorporation has taken place, the enzyme will either continue to incorporate nucleotides, causing a processive mistake, or a terminal event will occur and elongation will be arrested. Long and Accurate (LA) PCR combines a highly processive thermostable polymerase with a second thermostable polymerase that exhibits a 3'→5' exonucleolytic activity. This blend increases the length of amplification products by using the proofreading polymerase to repair terminal misincorporations. This repair allows the polymerase to resume elongating the growing DNA strand. AccuTaq LA Polymerase Mix combines Sigma's high quality Taq DNA polymerase with a small amount of a thermostable proofreading enzyme. The result is an enzyme mix that amplifies genomic targets in excess of 20-kb. Using a less complex template, such as bacterial genomic or viral DNA, amplifications of up to 20-kb or 40-kb, respectively, have been achieved. The fidelity of AccuTaq LA is 6.5 times greater than Taq DNA polymerase alone.

Reliable amplification of long DNA sequences requires: 1) effective denaturation of DNA template, 2) adequate extension times to produce large products and 3) protection of target DNA from damage by depurination. Effective denaturation is accomplished by the use of higher temperatures for shorter periods of time or by the use of co-solvents, such as dimethyl sulfoxide, In some cases, the extension times should be increased to greater than 20 minutes to accommodate the longer lengths of target DNA. The condition of the target DNA is critical. Depurination during cycling is minimized by use of buffers with a pH greater than 9.0 at 25°C.

### Reagents Provided

• AccuTaq LA DNA Polymerase, Product No. D 5553 5 units/µl in 20 mM Tris-HCl, pH 8.0, 100 mM KCl, 0.1 mM EDTA, 1 mM DTT, 0.5% Tween 20, 0.5% IGEPAL CA-630, 50% glycerol Provided as 125, 500, and 1500 units
• 10X Buffer for AccuTaq LA DNA Polymerase. Product No. B0174, 0.5 ml vial 500 mM Tris-HCl, 150 mM ammonium sulfate (pH 9.3, adjusted with KOH), 25 mM MgCl₂ 1% Tween 20 Provided as 3 vials/125 units, 3 vials/500 unit and 9 vials/1,500 units
• DMSO, Product No. D 8418 Provided as 1 ml

Materials and reagents Required but not Provided (Sigma Product Numbers have been given where appropriate)
- Deoxynucleotide Mix, Product No. D 7295
   10 mM dATP. 10 mM dCTP
   10 mM dGTP, 10 mM TTP
- Water, Product No. W 1754
- Mineral Oil, Product No. M 8662
- Primers
- DNA to be amplified
- Dedicated pipets
- PCR pipet tips
- 0.5 ml thin wall PCR microcentrifuge tubes
- Thermal cycler

### Precautions and Disclaimer

Sigma's AccuTaq LA Polymerase Mix is for laboratory use only; not for drug, household or other uses. Warning statements are included on the label or in the components section of this bulletin where applicable. In addition, when using radioactively labeled nucleic acids, standard procedures for safely handling radioactive materials should be followed.

4. The amplification parameters should be optimized for individual primers, template, and thermocycler. Suggested cycling parameters (based on in-house amplification of λ-globin gene cluster fragments from human genomic DNA):

| | |
|---|---|
| Initial denaturation | 98°C 30 sec |
| Denaturation | 94°C 5-15 sec |
| Annealing | 65°C 20 sec* |
| Extension | 68°C 20 min** |

| | |
|---|---|
| 30 cycles of amplification followed by a final 10 min extension at 68°C are recommended. Notes: *Oligonucleotides were between 21 bases (high G+C content) and 34 bases (high A+T content) in length. Melting temperatures of oligonucleotides used for amplification of genomic DNA were 62° -70°C. This was determined using the algorithm based upon nearest neighbor analysis of Rychlik and Rhoads (1989). " When amplifying templates 20 kb or greater, a 15 second auto-extension is suggested for cycles 16-30. Some thermal cyclers may not have this auto-extension function: increasing the extension time by 1-4 minute increments is recommended. | |

5. The amplified DNA can be evaluated by agarose gel electrophoresis and subsequent ethidium bromide staining.

### AMPLIFICATION PROCEDURE FOR LAMBDA DNA

The optimal conditions for the concentration of Taq DNA polymerase, template DNA, primers, and MgCl₂ will depend on the system being utilized. It may be necessary to determine the optimal conditions for each individual component.

**1. Add the following reagents to a thin-walled 500 µl PCR microcentrifuge tube:**

| Volume | Reagent | Final Concentration |
|---|---|---|
| 5 µl | 10X PCR Buffer | 1X |
| 2.5 µl | dNTP Mix (10 mM each) | 500 µM |
| 3 µl | Template DNA (2.5 ng/µl) | 0.15 ng/µl |
| 3 µl | Forward Primer (10 pmole/µl) | 600 nM |
| 3 µl | Reverse Primer (10 pmole/µl) | 600 nM |
| 33 µl | Water | - |
| 0.5µl | AccuTaq LA DNA Polymerase Mix | ____ 0.05 units/µl |

| | | |
|---|---|---|
| 50 µl Total Volume | | |

2. Mix gently and briefly centrifuge to collect all components to the bottom of the tube.

3. Add 50 µl of mineral oil to the top of each tube to prevent evaporation.

4. The amplification parameters should be optimized for individual primers, template, and thermal cycler. Suggested cycling parameters (based on in-house amplification of lambda DNA):

| | |
|---|---|
| Initial denaturation | 98°C 30 sec |
| Denaturation | 94°C 5.15 sec |
| Annealing | 65°C 20 sec⁺ |
| Extension | 68°C 20 min** |

| | |
|---|---|
| 30 cycles of amplification followed by a final 10 min extension at 68°C are recommended. Notes: -Oligonucleotides were between 21 bases (high G+C content) and 34 bases (high A+T content) in length. Melting temperatures of oligonucleotides used for amplification of genomic DNA were 62°-70°C. This was determined using the algorithm based upon nearest neighbor analysis of Rychlik and Rhoads (1989). ** When amplifying templates 20 kb or greater, a 15 second auto-extension is suggested for cycles 16-30. Some thermal cyclers may not have this auto-extension function; increasing the extension time by 1-4 minute increments is recommended. | |

5. The amplified DNA can be evaluated by agarose gel electrophoresis and subsequent ethidium bromide staining.

### References

Barnes, W.M. PCR amplification of up to 35-kb DNA with high fidelity and high yield from λ bacteriophage templates. Proc. Natl. Acad. Sci. USA 91:2216-2220 (1994)
Cheng, S., et al. Effective amplification of long targets from cloned inserts and human genomic DNA. Proc. Natl. Acad. Sci. USA 91:5695-5699 (1994)
Don, R. H., et al. "Touchdown" PCR to circumvent spurious priming during gene amplification. Nucl. Acids Res. 19:4008 (1991)
Erlich, H.A. (Ed.) PCR Technology: Principles and Applications for DNA Amplification, Stockton Press, New York (1989). (Product No. P 3551)
Frey, B. and Suppmann, B. Demonstration of the Expand™ PCR system's greater fidelity and higher yields with a lacl-based PCR fidelity assay. Biochemica 2:8-9 (1995)
Friezner-Degen, S. J., et al., J. Biol. Chem., 261: 6972-6984 (1986)
Griffin, H.G. and Griffin. A.M. (Eds.) PCR Technology: Current Innovations, CRC Press, Boca Raton, FL, 1994. (Product No. Z35, 749-9)
Innis, M.A., et al. (Eds.) PCR Strategies, Academic Press, New York (1995). (Product No. Z36, 445-2)
Innis, M., et al. (Eds.) PCR Protocols: A Guide to Methods and Applications, Academic Press, San Diego, California (1990). (Product No. P 8177)
McPherson. M.J. et al. (Eds.) PCR 2: A Practical Approach. IRL Press. New York (1995). (Product No. Z36. 238-7)
Nelson, et at. The fidelity of TaqPlus™ DNA Polymerase in PCR. Strategies Mol. Biol. 8:24-25 (1995)
Newton, C.R. (Ed.) PCR: Essential Data, John Wiley & Sons, New York (1995). (Product No. Z36. 491-6)
Roux, KH. Optimization and troubleshooting in PCR. PCR Methods Appl. 4:5185-5194 (1995)
Rychlik and Rhoads, Nucl. Acids Res. 17:8543-8551 (1989)
Saiki, R., PCR Technology: Principles and Applications for DNA Amplification, Stockton, New York (1989)
Sambrook, J, et al. Molecular Cloning: A Laboratory Manual, Second Edition. Cold Spring Harbor Laboratory Press, New York (1989). (Product No. M 3401)
†The PCR process is covered by patents owned by Hoffman-LaRoche, Inc.

### SEQUENCE LISTING:

GENERAL INFORMATION
   APPLICANT:
   NAME: Centeon Pharma GmbH
   LOCATION: Emil-von-Behring -Str. 76
   CITY: Marburg
   COUNTRY: Germany
   POSTAL CODE: 35041
   TELEPHONE: 06421-39-2069
   TELEFAX: 06421-39-4558
TITLE OF INVENTION: Labeled primer for use in and detection of target nucleic acids
NUMBER OF SEQUENCES: 6
   COMPUTER READABLE FORM:
   MEDIUM TYPE: Floppy disc
   COMPUTER: IBM PC compatible
   OPERATING SYSTEM: PC-DOS/MS-DOS
   SOFTWARE: WINWORD 6.0
INFORMATION FOR SEQ ID NO: 1:
   LENGTH: 21 bases
   TYPE: Nucleotid
   STRANDEDNESS: Single strand
   TOPOLOGY: linear
TYPE OF MOLECULE: Genome-DNA
HYPOTHETICAL: NO
ANTISENSE: NO
ORIGINAL SOURCE:
   ORGANISM: Hepatitis C Virus
POSITION IN GENOME:
   FEATURE: 5'-nontranslated region
SEQUENCE DESCRIPTION: SEQ ID NO: 1:
   ^{5'} GCGTCTAGCCATGGCGTTAGT ^{3'} 21
INFORMATION FOR SEQ ID NO: 2:
   LENGTH: 29 bases
   TYPE: Nucleotid
   STRANDEDNESS: Single strand
   TOPOLOGY: linear
TYPE OF MOLECULE: Genome-DNA
HYPOTHETICAL: NO
ANTISENSE: NO
ORIGINAL SOURCE:
   ORGANISM: Hepatitis C Virus
**POSITION IN GENOME:**
   FEATURE: 5'-nontranslated region
SEQUENCE DESCRIPTION: SEQ ID NO: 2:
   ⁵' CCACAAGGCCTTTCGCGACCCAACTTACT 3' 29
INFORMATION FOR SEQ ID NO: 3:
   LENGTH: 29 bases
   TYPE: Nucleotid
   STRANDEDNESS: Single strand
   TOPOLOGY: linear
TYPE OF MOLECULE: Genome-DNA
HYPOTHETICAL: NO
ANTISENSE: NO
ORIGINAL SOURCE:
   ORGANISM: Hepatitis C Virus
POSITION IN GENOME:
   FEATURE: 5'-nontranslated region
SEQUENCE DESCRIPTION: SEQ ID NO: 3:
   5' REPORTER- CCACAAGGCCTTTCGCGACCCAACTTACT-QUENCHER 3' 29
INFORMATION FOR SEQ ID NO: 4:
   LENGTH: 22 bases
   TYPE: Nucleotid
   STRANDEDNESS: Single strand
   TOPOLOGY: linear
TYPE OF MOLECULE: Genome-DNA
HYPOTHETICAL: NO
ANTISENSE: NO
ORIGINAL SOURCE:
   ORGANISM: Hepatitis B Virus
POSITION IN GENOME:
   FEATURE: core gene
SEQUENCE DESCRIPTION: SEQ ID NO: 4:
   ^{5'} AATCCACACTCCGAAAGACACC^{3'} 22
INFORMATION FOR SEQ ID NO: 5:
   LENGTH: 20 bases
   TYPE: Nucleotid
   STRANDEDNESS: Single strand
   TOPOLOGY: linear
TYPE OF MOLECULE: Genome-DNA
HYPOTHETICAL: NO
ANTISENSE: NO
ORIGINAL SOURCE:
   ORGANISM: Hepatitis B Virus
POSITION IN GENOME:
   FEATURE: precore region
SEQUENCE DESCRIPTION: SEQ ID NO: 5:
   5' GCCTCCAAGCTGTGCCTTGG' 3' 20
INFORMATION FOR SEQ ID NO: 6:
   LENGTH: 24 bases
   TYPE: Nucleotid
   STRANDEDNESS: Single strand
   TOPOLOGY: linear
TYPE OF MOLECULE: Genome-DNA
HYPOTHETICAL: NO
ANTISENSE: NO
ORIGINAL SOURCE:
   ORGANISM: Hepatitis B Virus
POSITION IN GENOME:
   FEATURE: precore region
SEQUENCE DESCRIPTION: SEQ ID NO: 6:
   ^{5'} REPORTER-GCCTCCAAGCTGTGCCTTGGTGAA-QUENCHER ^{3'} 24

## Claims

1. A process for the detection of the presence of a target nucleic acid in a sample comprising single-stranded DNA said process comprising
(a) contacting said sample with a forward and/or reverse primer,
wherein at least one primer carries a label or part of a label system in the 3' terminal portion of the primer and wherein said primer is selected such that at least one, preferably at least the last two to five or more nucleotides at the 3' end of the primer are not complementary to the nucleic acid sequence to be detected
to create a mixture of duplexes during hybridization conditions with the forward and/or said reverse primer annealed to complementary DNA sequences each of the target nucleic acid in case of its presence;
(b) maintaining the mixture of step (a) with a template-dependent nucleic acid polymerase having a 3' to 5' proofreading activity or a mixture of enzymes having such proofreading activity under conditions sufficient to permit the 3' to 5' nuclease activity of said polymerase or mixture of enzymes to cleave off the 3' mismatched portion of the annealed primer thereby releasing label or part of a label system;
(c) detecting and/or measuring the release of label or part of a label system.

2. A process according to claim 1 wherein the target nucleic acid is amplified and thereby more label or part of a label system is released.

3. A process according to claim 2 in which the release of the label is directly proportional to the amount of amplified DNA

4. A process according to claim 2 or claim 3 wherein the amplification is performed by PCR, optionally by RT-PCR in case the target nucleic acid is RNA.

5. A process according to one of the preceding claims, wherein the label is a reporter-quencher molecule pair linked to the 3' and 5' terminal regions of the forward and/or the reverse primer.

6. A process according to one of the preceding claims wherein the reporter molecule, is on the mismatched 3' part of the primer and the quencher molecule on the matched part of said primer in suitable distance of the reporter molecule.

7. A process according to claim 5 wherein the quencher molecule is on the mismatched 3' part of the primer and the reporter molecule on the matched part of said primer in suitable distance of the reporter molecule, preferably at the 5' end.

8. A process according to one of the preceding claims wherein the said nucleic acid polymerase or said mixture of enzymes are thermostable.

9. A process according to one of the preceding claims wherein a multiplicity of nucleic acid targets is detected simultaneously by selection of corresponding primers wherein at least one primer for each target carries a label or part of a label system in the 3' terminal portion of the primer.

10. A process according to one of the preceding claims wherein the sample is animal or human body fluid, preferably plasma.

11. A kit for the detection of a target nucleic acid in a sample comprising labeled primers used in the processes of claims 1 to 10 and a suitable nucleic acid polymerase having a 3' to 5' proofreading activity or a mixture of enzymes having such proofreading activity.

12. A kit as claimed in claim 10 wherein the primers detect target viral nucleic acids.

## Patentansprüche

1. Verfahren zur Detektion des Vorhandenseins einer Targetnukleinsäure in einer einzelsträngige DNA umfassenden Probe, welches Verfahren umfaßt
(a) das Inkontaktbringen der Probe mit einem Vorwärts- und/oder Rückwärts-Primer,
wobei wenigstens ein Primer eine Markierung oder einen Teil eines Markierungssystems im 3'-terminalen Abschnitt des Primers aufweist,
und worin der Primer derart ausgewählt ist, daß wenigstens eines, vorzugsweise wenigstens die letzten zwei bis fünf oder mehr Nukleotide am 3'-Ende des Primers zur zu detektierenden Nukleinsäuresequenz nicht komplementär sind,
um ein Gemisch von Duplexen während der Hybridisierungsbedingungen mit dem Vorwärts- und/oder Rückwärts-Primer auszubilden, aneliert an komplementäre DNA-Sequenzen jeder Targetnukleinsäure im Fall ihres Vorliegens;
(b) Halten des Gemisches aus Schritt (a) mit einer Template-abhängigen Nukleinsäure-Polymerase mit einer 3' nach 5'-Korrekturleseaktivität oder einem Gemisch von Enzymen mit einer derartigen Korrekturleseaktivität unter Bedingungen, die ausreichend sind, die 3' nach 5'-Nuklease-Aktivität der Polymerase oder des Gemisches von Enzymen zu ermöglichen, um den unpassenden 3'-Abschnitt des anelierten Primers abzuspalten, wodurch eine Markierung oder ein Teil eines Markierungssystems freigesetzt wird;
(c) das Detektieren und/oder Messen der Freisetzung der Markierung oder des Teils eines Markierungssystems.

2. Verfahren nach Anspruch 1, worin die Targetnukleinsäure amplifiziert wird und **dadurch** mehr von der Markierung oder dem Teil eines Markierungssystems freigesetzt wird.

3. Verfahren nach Anspruch 2, worin die Freisetzung der Markierung direkt proportional zur Menge an amplifizierter DNA ist.

4. Verfahren nach Anspruch 2 oder Anspruch 3, worin die Amplifikation durch PCR, wahlweise durch RT-PCR im Fall, daß die Targetnukleinsäure RNA ist, durchgeführt wird.

5. Verfahren nach einem der vorstehenden Ansprüche, worin die Markierung ein Reporter-Quencher-Molekülpaar ist, welches an die 3' und 5' terminalen Regionen des Vorwärts- und/oder Rückwärts-Primers gebunden ist.

6. Verfahren nach einem der vorstehenden Ansprüche, worin das Reporter-Molekül am unpassenden 3'-Teil des Primers vorliegt und das Quencher-Molekül am passenden Teil des Primers in geeignetem Abstand zum Reporter-Molekül vorliegt.

7. Verfahren nach Anspruch 5, worin das Quencher-Molekül am unpassenden 3'-Teil des Primers vorliegt und das Reporter-Molekül am passenden Teil des Primers in geeigneter Entfernung zum Reportermolekül, vorzugsweise am 5'-Ende, vorliegt.

8. Verfahren nach einem der vorstehenden Ansprüche, worin die Nukleinsäurepolymerase oder das Enzymgemisch thermostabil ist.

9. Verfahren nach einem der vorstehenden Ansprüche, worin eine Mehrzahl von Nukleinsäuretargets durch Auswahl der entsprechenden Primer gleichzeitig detektiert wird, wobei wenigstens ein Primer für jedes Target eine Markierung oder einen Teil eines Markierungssystems im 3'-Terminalabschnitt des Primers umfaßt.

10. Verfahren nach einem der vorstehenden Ansprüche, worin die Probe ein tierisches oder humanes Körperfluid, vorzugsweise Plasma ist.

11. Kit zur Detektion einer Targetnukleinsäure in einer Probe, umfassend markierte Primer, wie sie in den Verfahren der Ansprüche 1 bis 10 verwendet werden, und eine geeignete Nukleinsäurepolymerase mit einer 3' nach 5'-Korrekturleseaktivität oder ein Gemisch von Enzymen mit einer derartigen Korrekturleseaktivität.

12. Kit nach Anspruch 10, worin die Primer virale Targetnukleinsäuren detektieren.

## Revendications

1. Procédé pour la détection de la présence d'un acide nucléique cible dans un échantillon comprenant un ADN monocaténaire, ledit procédé comprenant :
(a) la mise en contact dudit échantillon avec une amorce directe et/ou inverse,
au moins une amorce portant un marqueur ou une partie d'un système marqueur dans la portion 3' terminale de l'amorce,
et ladite amorce étant sélectionnée de sorte qu'au moins un, de préférence au moins les deux à cinq ou plus derniers nucléotides à l'extrémité 3' de l'amorce ne sont pas complémentaires de la séquence d'acide nucléique à détecter pour créer un mélange de duplex pendant les conditions d'hybridation avec ladite amorce directe et/ou inverse hybridée à chacune des séquences d'ADN complémentaire de l'acide nucléique cible dans le cas de sa présence ;
(b) le maintien du mélange de l'étape (a) avec une polymérase d'acide nucléique dépendante de la matrice ayant une activité de correction de 3' en 5' ou un mélange d'enzymes ayant cette activité de correction dans des conditions suffisantes pour permettre l'activité nucléase de 3' en 5' de ladite polymérase ou dudit mélange d'enzymes afin de cliver la portion mésappariée en 3' de l'amorce hybridée en libérant ainsi le marqueur ou une partie d'un système marqueur ;
(c) la détection et/ou la mesure de la libération du marqueur ou d'une partie du système de marqueur.

2. Procédé selon la revendication 1, dans lequel l'acide nucléique cible est amplifié, et ainsi davantage de marqueur ou de partie d'un système marqueur est libéré.

3. Procédé selon la revendication 2, dans lequel la libération du marqueur est directement proportionnelle à la quantité d'ADN amplifié.

4. Procédé selon la revendication 2 ou la revendication 3, dans lequel l'amplification est effectuée par PCR, éventuellement par RT-PCR dans le cas où l'acide nucléique est un ARN.

5. Procédé selon l'une quelconque des revendications précédentes, dans lequel le marqueur est une paire de molécules rapporteur-quencheur liée aux régions terminales 3' et 5' de l'amorce directe et/ou inverse.

6. Procédé selon l'une quelconque des revendications précédentes, dans lequel la molécule rapporteur est sur la partie mésappariée en 3' de l'amorce et la molécule quencheur sur la partie appariée de ladite amorce à une distance appropriée de la molécule rapporteur.

7. Procédé selon la revendication 5, dans lequel la molécule quencher est sur la partie mésappariée en 3' de l'amorce et la molécule rapporteur sur la partie appariée de ladite amorce à une distance appropriée de la molécule rapporteur, de préférence à l'extrémité 5'.

8. Procédé selon l'une quelconque des revendications précédentes, dans lequel ladite polymérase d'acide nucléique ou ledit mélange d'enzymes est thermostable.

9. Procédé selon l'une quelconque des revendications précédentes, dans lequel une multiplicité de cibles d'acides nucléiques est détectée simultanément par sélection d'amorces correspondantes, parmi lesquelles au moins une amorce pour chaque cible porte un marqueur ou une partie d'un système marqueur dans la portion 3' terminale de l'amorce.

10. Procédé selon l'une quelconque des revendications précédentes, dans lequel l'échantillon est un liquide corporel animal ou humain, de préférence le plasma.

11. Kit pour la détection d'un acide nucléique dans un échantillon comprenant des amorces marquées utilisées dans les procédés des revendications 1 à 10 et une polymérase d'acide nucléique ayant une activité de correction de 3' en 5' ou un mélange d'enzymes ayant cette activité de correction.

12. Kit tel que revendiqué dans la revendication 10, dans lequel les amorces détectent des acides nucléiques viraux cibles.


### SEQUENCE LISTING:

**(1) GENERAL INFORMATION:**
   (i) APPLICANT:
      (A) NAME: Centeon Pharma GmbH
      (B) STREET: Emil-von-Behring-Strasse 76
      (C) CITY: Marburg
      (E) COUNTRY: Germany
      (F) POSTAL CODE: 35041
   (ii) TITLE OF INVENTION: Labeled primer for use in and detection of target nucleic acids
   (iii) NUMBER OF SEQUENCES: 6
   (iv) CORRESPONDENCE ADDRESS:
      (A) ADDRESSEE: Centeon Pharma GmbH
      (B) STREET: P.O. Box 12 30
      (C) CITY: Marburg
      (E) COUNTRY: Germany
      (F) POSTAL CODE: 35002
   (v) COMPUTER READABLE FORM:
      (A) MEDIUM TYPE: Floppy disc
      (B) COMPUTER: IBM PC compatible
      (C) OPERATING SYSTEM: PC-DOS/MS-DOS
      (D) SOFTWARE: Microsoft Word 6.0
   (vi) CURRENT APPLICATION DATA:
      (A) APPLICATION NUMBER: 98123569.0
      (B) FILING DATE: 10.12.98
   (vii) PRIOR APPLICATION DATA:
      (A) APPLICATION NUMBER: 19755642.6 (DE)
      (B) FILING DATE: 15.12.97

      (A) APPLICATION NUMBER: 19806850.6 (DE)
      (B) FILING DATE: 19.02.98

      (A) APPLICATION NUMBER: 19834913.0 (DE)
      (B) FILING DATE: 03.08.98
   (ix) TELECOMMUNICATION INFORMATION:
      (A) TELEPHONE: 06421-39-2069
      (B) TELEFAX: 06421-39-4558
**(2) INFORMATION FOR SEQ ID NO: 1:**
   (i) SEQUENCE CHARACTERISTICS:
      (A) LENGTH: 21 bases
      (B) TYPE: Nucleotid
      (C) STRANDEDNESS: Single strand
      (D) TOPOLOGY: linear
   (ii) MOLECULE TYP: Genome-DNA
   (iii) HYPOTHETICAL: No
   (iv) ANTISENSE: No
   (vi) ORIGINAL SOURCE:
      (A) ORGANISM: Hepatitis C Virus
   (ix) FEATURES:
      (B) LOCATION: 5'-nontranslated region
   **(xi) SEQUENCE DESCRIPTION: SEQ ID NO: 1:**
      GCGTCTAGCCATGGCGTTAGT 21
**(2) INFORMATION FOR SEQ ID NO: 2:**
   (i) SEQUENCE CHARACTERISTICS:
      (A) LENGTH: 29 bases
      (B) TYPE: Nucleotid
      (C) STRANDEDNESS: Single strand
      (D) TOPOLOGY: linear
   (ii) MOLECULE TYPE: Genome-DNA
   (iii) HYPOTHETICAL: No
   (iv) ANTISENSE: No
   (vi) ORIGINAL SOURCE:
      (A) ORGANISM: Hepatitis C Virus
   (ix) FEATURES:
      (B) LOCATION: 5'-nontranslated region
   **(xi) SEQUENCE DESCRIPTION: SEQ ID NO: 2:**
      CCACAAGGCCTTTCGCGACCCAACTTACT 29
**(2) INFORMATION FOR SEQ ID NO: 3:**
   (i) SEQUENCE CHARACTERISTICS:
      (A) LENGTH: 29 bases
      (B) TYPE: Nucleotid
      (C) STRANDEDNESS: Single strand
      (D) TOPOLOGY: linear
   (ii) MOLECULE TYPE: Genome-DNA
   (iii) HYPOTHETICAL: No
   (iv) ANTISENSE: No
   (vi) ORIGINAL SOURCE:
      (A) ORGANISM: Hepatitis C Virus
   (ix) FEATURES:
      (B) LOCATION: 5'-nontranslated region
   **(xi) SEQUENCE DESCRIPTION: SEQ ID NO:** 3:
      CCACAAGGCCTTTCGCGACCCAACTTACT 29
**(2) INFORMATION FOR SEQ ID NO: 4:**
   (i) SEQUENCE CHARACTERISTICS:
      (A) LENGTH: 22 bases
      (B) TYPE: Nucleotid
      (C) STRANDEDNESS: Single strand
      (D) TOPOLOGY: linear
   (ii) MOLECULE TYPE: Genome-DNA
   (iii) HYPOTHETICAL: No
   (iv) ANTISENSE: No
   (vi) ORIGINAL SOURCE:
      (A) ORGANISM: Hepatitis B Virus
   (ix) FEATURES:
      (B) LOCATION: core gene
   **(xi) SEQUENCE DESCRIPTION: SEQ ID NO: 4:**
      AATCCACACTCCGAAAGACACC 22
**(2) INFORMATION FOR SEQ ID NO: 5:**
   (i) SEQUENCE CHARACTERISTICS:
      (A) LENGTH: 20 bases
      (B) TYPE: Nucleotid
      (C) STRANDEDNESS: Single strand
      (D) TOPOLOGY: linear
   (ii) MOLECULE TYPE: Genome-DNA
   (iii) HYPOTHETICAL: No
   (iv) ANTISENSE: No
   (vi) ORIGINAL SOURCE:
      (A) ORGANISM: Hepatitis B Virus
   (ix) FEATURES:
      (B) LOCATION: precore region
   **(xi) SEQUENCE DESCRIPTION: SEQ ID NO: 5:**
      GCCTCCAAGCTGTGCCTTGG 20
**(2) INFORMATION FOR SEQ ID NO: 6:**
   (i) SEQUENCE CHARACTERISTICS:
      (A) LENGTH: 24 bases
      (B) TYPE: Nucleotid
      (C) STRANDEDNESS: Single strand
      (D) TOPOLOGY: linear
   (ii) MOLECULE TYPE: Genome-DNA
   (iii) HYPOTHETICAL: No
   (iv) ANTISENSE: No
   (vi) ORIGINAL SOURCE:
      (A) ORGANISM: Hepatitis B Virus
   (ix) FEATURES
      (B) LOCATION: precore region
   **(xi) SEQUENCE DESCRIPTION: SEQ ID NO: 6:**
      GCCTCCAAGCTGTGCCTTGGTGAA 24